# FASCICULE DE BREVET EUROPEEN

(11) **EP 0 888 792 B1**
(45) Date de publication et mention de la délivrance du brevet: **21.04.2004**
(21) Numéro de dépôt: 98401592.5
(22) Date de dépôt: 26.06.1998
(51) Int. Cl.: A61M 16/04

(54) **Sonde d'aspiration endotrachéale pour patient sous ventilation artificielle**
Endotrachealer Absaugtubus für Patienten unter künstlicher Beatmung
Suction endotracheal tube for use in a patient under artificial ventilation

(30) Priorité: 30.06.1997 FR 9708187
(43) Date de publication de la demande: 07.01.1999
(73) Titulaire: Boussignac, Georges, 92160 Antony (FR)
(72) Inventeur: Boussignac, Georges, 92160 Antony (FR)
(74) Mandataire: Bonnetat, Christian

(56) Documents cités:
- US-A- 5 029 580
- US-A- 5 125 893
- US-A- 5 140 983
- US-A- 5 167 622

## Description

La présente invention concerne une sonde d'aspiration endotrachéale pour un patient sous ventilation artificielle.

De telles sondes sont décrites dans les documents US-A-5 140 983 et US-A-5 029 580.

On sait qu'un patient sous ventilation artificielle est relié à un appareil de respiration artificielle par l'intermédiaire d'un tube de ventilation placé dans sa trachée et alimenté en gaz respiratoire par ledit appareil. Un tel patient sécrète des mucosités pulmonaires qu'il est nécessaire d'éliminer plusieurs fois par jour. Aussi, on connaît déjà des sondes à aspirer ces mucosités pulmonaires. En fonction du mode de réalisation du tube de ventilation, une telle sonde peut être introduite dans la trachée soit en remplacement dudit tube de ventilation, soit à travers celui-ci et la ventilation artificielle soit doit être interrompue, soit au contraire peut être maintenue pendant l'aspiration des mucosités.

Cependant, même dans le cas le plus favorable où la ventilation artificielle est maintenue pendant l'aspiration des mucosités, il existe des risques d'hypoxie non négligeables pour le patient, pouvant entraîner un ralentissement, et même un arrêt, cardiaque et dus au fait qu'un respirateur artificiel n'insuffle du gaz respiratoire que pendant environ un tiers du temps. Ainsi, pendant les autres deux tiers du temps, l'aspiration des mucosités pulmonaires peut entraîner un collapsus pulmonaire.

La présente invention a pour objet d'éliminer totalement les risques d'hypoxie, lors de l'aspiration des mucosités pulmonaires d'un patient sous ventilation artificielle.

A cette fin, selon l'invention, la sonde d'aspiration endotrachéale de mucosités pulmonaires pour un patient sous ventilation artificielle relié à un appareil de respiration artificielle par l'intermédiaire d'un tube de ventilation placé dans sa trachée, ladite sonde d'aspiration étant prévue pour être reliée à des moyens d'aspiration et pour être introduite dans ledit tube de ventilation, est remarquable en ce qu'elle comporte un canal de soufflage permanent d'un gaz respiratoire sous pression et en ce que l'orifice distal dudit canal de soufflage est latéral et suffisamment éloigné de l'extrémité distale de ladite sonde pour assurer que l'orifice distal se trouve toujours en regard de la paroi interne dudit tube de ventilation, lorsque ladite sonde d'aspiration est en service à l'intérieur dudit tube de ventilation.

Ainsi, pendant l'aspiration des mucosités pulmonaires, du gaz respiratoire est insufflé dans la trachée du patient, de sorte qu'il ne peut en résulter un collapsus pulmonaire. Par ailleurs, du fait que l'orifice distal du canal de soufflage se trouve en regard de la paroi interne du tube de ventilation, ce gaz de soufflage ne peut blesser les muqueuses trachéales et bronchiques.

Pour obtenir ce résultat, il est avantageux que, dans le cas d'un adulte, la distance comprise entre l'orifice distal du canal de soufflage et l'extrémité distale de la sonde d'aspiration soit au moins égale à 10 cm, et de préférence au moins approximativement égale à 15 cm. Si la sonde est destinée à un enfant en bas âge, cette distance peut être au moins égale à 2 cm.

Il est avantageux de prévoir, dans ladite sonde d'aspiration, un canal de prise de pression pulmonaire débouchant au voisinage de l'extrémité distale de la sonde d'aspiration et d'utiliser la pression dans ledit canal de prise de pression pour ajuster le débit du gaz respiratoire de soufflage, afin d'éviter le collapsus pulmonaire.

Le gaz de soufflage peut par exemple être de l'oxygène.

Dans un mode de réalisation avantageux, le canal de soufflage et/ou le canal de prise de pression sont pratiqués dans la paroi de ladite sonde d'aspiration.

Avantageusement, la pression du gaz respiratoire de soufflage est choisie au plus égale à 3,5 bars, tandis que la dépression à l'extrémité distale de la sonde d'aspiration est de l'ordre de quelques centaines de millibars.

Les figures du dessin annexé feront bien comprendre comment l'invention peut être réalisée. Sur ces figures, des références identiques désignent des éléments semblables.

La figure 1 est une vue en coupe longitudinale schématique, illustrant la sonde d'aspiration endotrachéale conforme à la présente invention, en place dans un tube de ventilation.

La figure 2 est une coupe selon la ligne II-II de la figure 1.

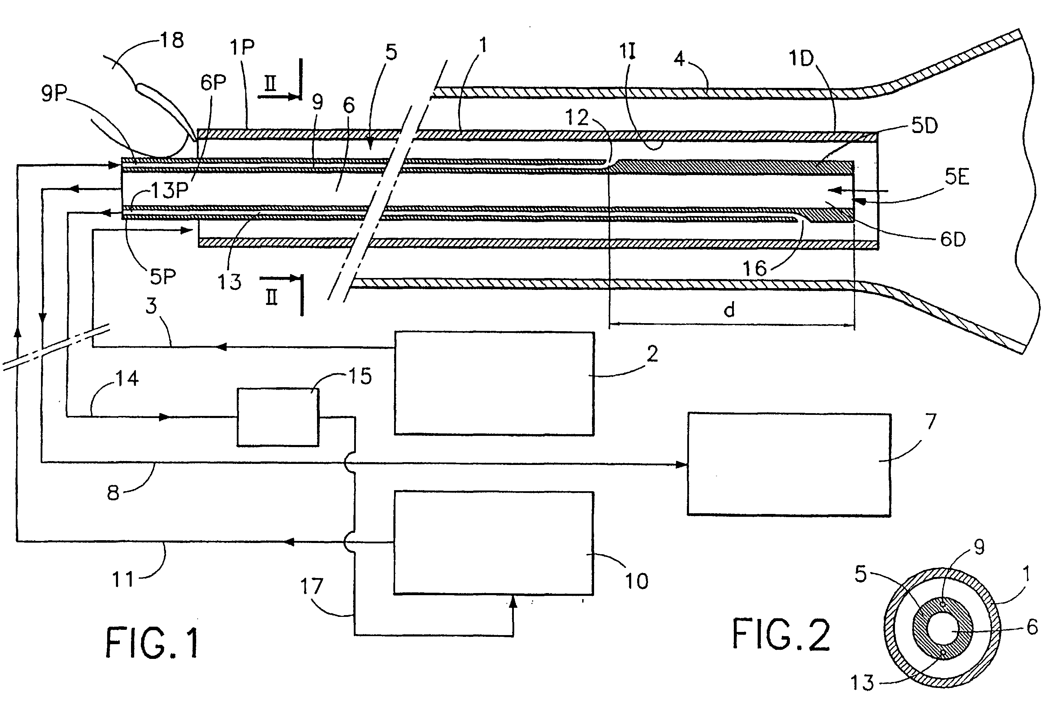

Sur la figure 1, on a représenté schématiquement un tube de ventilation 1, relié à un respirateur artificiel 2 par une liaison 3 et destiné à ventiler un patient, dont seule la trachée 4 est représentée. A cet effet, la partie distale 1D dudit tube de ventilation 1 est logée dans ladite trachée 4, tandis que la partie proximale 1P du tube de ventilation 1 sort de la bouche du patient (non représentée), est extérieure à celui-ci et est reliée audit respirateur artificiel 2 par la liaison 3.

Dans le tube de ventilation 1, peut être introduite une sonde 5 pour l'aspiration des mucosités pulmonaires du patient. La sonde 5 comporte une lumière longitudinale centrale 6, dont la partie proximale 6P est reliée, par une liaison 8, à un aspirateur 7. Celui-ci crée, à l'extrémité distale 5E de ladite sonde 5, une dépression par exemple égale à quelques centaines de millibars.

La sonde d'aspiration 5 est pourvue d'un premier canal longitudinal 9, par exemple réalisé dans l'épaisseur de sa paroi, dont la partie proximale 9P est reliée à une source 10 de gaz respirable sous pression par une liaison 11. Le canal longitudinal 9 débouche dans la paroi de la sonde 5 par un orifice latéral 12, pratiqué dans la partie distale 5D de ladite sonde, mais à une distance d de l'extrémité distale 5E de celle-ci. Si la sonde 5 est destinée à un adulte, la distance d peut être de l'ordre de 10 cm à 15 cm. La source 10 adresse au premier canal longitudinal 9 un gaz de soufflage, par exemple à base d'oxygène, de sorte qu'un jet de gaz respirable, permanent pendant l'aspiration, sort de l'orifice latéral 12. La pression de ce jet de gaz respirable est au plus égale à 3,5 bars.

La sonde d'aspiration 5 est de plus pourvue d'un second canal longitudinal 13, par exemple réalisé dans l'épaisseur de sa paroi, dont la partie proximale 13P est reliée, par une liaison 14, à un capteur de pression 15. Le canal longitudinal 13 débouche dans la paroi de la sonde 5 par un orifice latéral 16, pratiqué dans la partie distale 5D de la sonde 5, au voisinage de ladite extrémité distale 5E.

Le capteur de pression 15 est relié à la source 10 par une liaison 17.

La sonde d'aspiration 5 est par exemple tenue à la main par un opérateur (non représenté) dont les doigts 18 saisissent l'extrémité proximale 5P de ladite sonde.

Sur la figure 1, la sonde d'aspiration 5 est représentée déjà en place dans le tube de ventilation artificielle 1.

Elle est tenue à son extrémité proximale 5P par les doigts 18 dudit opérateur, lesdits doigts butant contre l'extrémité proximale 1P du tube endotrachéal 1, ce qui définit l'enfoncement maximal de ladite sonde 5 dans ledit tube 1.

Même dans cette position d'enfoncement maximal, l'orifice de soufflage 12 se trouve en regard de la paroi interne 1I du tube 1, de sorte que le jet permanent du gaz de soufflage sortant dudit orifice 12 frappe cette paroi interne et est diffusé par le tube 1 vers les poumons du patient, sans possibilité de lésion des muqueuses trachéales et bronchiques.

La pression du gaz respiratoire dans les poumons du patient est prélevée par l'orifice 16 et transmise par le canal 13 et la liaison 14 au capteur de pression 15 qui, par la liaison 17, peut commander le débit de la source 10 pour l'ajuster à une valeur telle que toute hypoxie est évitée.

Ainsi, il est toujours possible d'optimiser la pression de gaz respiratoire dans les poumons du patient pendant l'aspiration des mucosités à travers la lumière 6 de la sonde 5, par l'intermédiaire de l'aspirateur 7 et de la liaison 8.

On conçoit aisément que la sonde d'aspiration 5 peut être facilement introduite dans le tube 1 (figure 1) ou sortie de celui-ci.

## Revendications

1. Sonde (5) d'aspiration endotrachéale de mucosités pulmonaires pour un patient sous ventilation artificielle relié à un appareil (2) de respiration artificielle par l'intermédiaire d'un tube de ventilation (1) placé dans sa trachée (14), ladite sonde d'aspiration (5) étant prévue pour être reliée à des moyens d'aspiration (7) et pour être introduite dans ledit tube de ventilation (1) et comportant un canal (9) de soufflage permanent d'un gaz respiratoire sous pression,
**caractérisée en ce que** l'orifice distal (12) dudit canal de soufflage (9) est latéral et suffisamment éloigné de l'extrémité distale (5E) de ladite sonde d'aspiration (5) pour assurer que l'orifice distal (12) se trouve toujours en regard de la paroi interne (1I) dudit tube de ventilation (1), lorsque ladite sonde d'aspiration (5) est en service à l'intérieur dudit tube de ventilation (1).

2. Sonde d'aspiration selon la revendication 1, destinée à un adulte,
**caractérisée en ce que** la distance (d) entre l'orifice distal (12) du canal de soufflage (9) et l'extrémité distale (5E) de ladite sonde d'aspiration est égale à au moins 10 cm.

3. Sonde d'aspiration selon la revendication 2,
**caractérisée en ce que** ladite distance (d) est au moins approximativement égale à 15 cm.

4. Sonde d'aspiration selon la revendication 1, destinée à un enfant en bas âge,
**caractérisée en ce que** la distance (d) entre l'orifice distal (12) du canal de soufflage (9) et l'extrémité distale (5E) de ladite sonde d'aspiration est égale à au moins 2 cm.

5. Sonde d'aspiration selon l'une des revendications 1 à 4,
**caractérisée en ce qu'**elle comporte un canal (13) de prise de pression pulmonaire débouchant (en 16) au voisinage de l'extrémité distale (5E) de ladite sonde d'aspiration (5).

6. Sonde d'aspiration selon la revendication 5,
**caractérisée en ce que** la pression dans ledit canal (13) de prise de pression pulmonaire commande le débit du gaz respiratoire de soufflage.

7. Sonde d'aspiration selon l'une quelconque des revendications 1 à 6,
**caractérisée en ce que** ledit canal de soufflage (9) est pratiqué dans la paroi de ladite sonde (5).

8. Sonde d'aspiration selon l'une quelconque des revendications 5 à 7,
**caractérisée en ce que** ledit canal de prise de pression (13) est pratiqué dans la paroi de ladite sonde (5).

## Claims

1. Endotracheal aspiration probe (5) for aspirating pulmonary mucus from a patient under artificial ventilation, connected to an artificial respiration apparatus (2) by way of a ventilation tube (1) placed in his trachea (14) , said aspiration probe (5) being intended to be connected to aspiration means (7) and to be introduced into said ventilation tube (1) and including a channel (9) for permanent blowing of a respiratory gas under pressure, **characterized in that** the distal orifice (12) of said blowing channel (9) is lateral and sufficiently distant from the distal end (5E) of said aspiration probe (5) to guarantee that the distal orifice (12) is always situated opposite the inner wall (1I) of said ventilation tube (1), when said aspiration probe (5) is in use inside said ventilation tube (1).

2. Aspiration probe according to Claim 1, intended for an adult, **characterized in that** the distance (d) between the distal orifice (12) of the blowing channel (9) and the distal end (5E) of said aspiration probe is equal to at least 10 cm.

3. Aspiration probe according to Claim 2, **characterized in that** said distance (d) is at least approximately equal to 15 cm.

4. Aspiration probe according to Claim 1, intended for a young child, **characterized in that** the distance (d) between the distal orifice (12) of the blowing channel (9) and the distal end (5E) of said aspiration probe is equal to at least 2cm.

5. Aspiration probe according to one of Claims 1 to 4, **characterized in that** it includes a channel (13) which is used for measuring pulmonary pressure and opens out (at 16) in the vicinity of the distal end (5E) of said aspiration probe (5).

6. Aspiration probe according to Claim 5, **characterized in that** the pressure in said channel (13) for measuring pulmonary pressure controls the flow rate of the respiratory gas being blown.

7. Aspiration tube according to any one of Claims 1 to 6, **characterized in that** said blowing channel (9) is formed in the wall of said probe (5).

8. Aspiration tube according to any one of Claims 5 to 7, **characterized in that** said channel (13) for measuring pressure is formed in the wall of said probe (5).

## Patentansprüche

1. Endotracheale Sonde (5) zum Absaugen des Lungenschleims für einen Patienten unter künstlicher Ventilation, der unter Zwischenschaltung eines in seiner Luftröhre (4) befindlichen Ventilationsschlauchs (1) mit einem Gerät (2) zur künstlichen Beatmung verbunden ist, wobei die Absaugsonde (5) dazu vorgesehen ist, mit Absaugmitteln (7) verbunden zu werden und in den Ventilationsschlauch (1) eingeführt zu werden, umfassend einen Kanal (9) zum ständigen Einblasen eines unter Druck stehenden Atemgases,
**dadurch gekennzeichnet, dass** sich die distale Öffnung (12) des Einblaskanals (9) seitlich und ausreichend weit entfernt vom distalen Ende (5E) der Absaugsonde (5) befindet, um sicherzustellen, dass sich die distale Öffnung (12) immer gegenüber der Innenwand (1I) des Ventilationsschlauchs (1) befindet, wenn die Absaugsonde (5) im Inneren des Ventilationsschlauchs (1) in Betrieb ist.

2. Absaugsonde nach Anspruch 1, bestimmt für einen Erwachsenen, **dadurch gekennzeichnet, dass** die Entfernung (d) zwischen der distalen Öffnung (12) des Einblaskanals (9) und dem distalen Ende (5E) der Absaugsonde mindestens gleich 10 cm beträgt.

3. Absaugsonde nach Anspruch 2, **dadurch gekennzeichnet, dass** die Entfernung (d) mindestens etwa gleich 15 cm beträgt.

4. Absaugsonde nach Anspruch 1, bestimmt für ein Kind mit geringem Alter, **dadurch gekennzeichnet, dass** die Entfernung (d) zwischen der distalen Öffnung (12) des Einblaskanals (9) und dem distalen Ende (5E) der Absaugsonde mindestens gleich 2 cm beträgt.

5. Absaugsonde nach einem der Ansprüche 1 bis 4, **dadurch gekennzeichnet, dass** sie einen Kanal (13) zur Aufnahme des Lungendrucks umfasst, der (bei 16) in der Nähe des distalen Endes (5E) der Absaugsonde (5) mündet.

6. Absaugsonde nach Anspruch 5, **dadurch gekennzeichnet, dass** der Druck im Kanal (13) zur Aufnahme des Lungendrucks den Durchsatz des Einblas-Atemgases steuert.

7. Absaugsonde nach einem der Ansprüche 1 bis 6, **dadurch gekennzeichnet, dass** der Einblaskanal (9) in der Wand der Sonde (5) gebildet ist.

8. Absaugsonde nach einem der Ansprüche 5 bis 7, **dadurch gekennzeichnet, dass** der Kanal zur Aufnahme des Drucks (13) in der Wand der Sonde (5) gebildet ist.
